# EUROPEAN PATENT SPECIFICATION

(11) **EP 2 371 349 B1**
(45) Date of publication and mention of the grant of the patent: **29.08.2012**
(21) Application number: 11172216.1
(22) Date of filing: 10.02.2004
(51) Int. Cl.: A61K 8/36, A61K 8/92, A61Q 19/10, A61K 8/31, A61Q 19/00, G01N 33/483

(54) **Method of detection of ashen skin**
Methode zur Detektion von Aschehaut
Méthode de détection d'une peau cendrée

(30) Priority: 10.02.2003 US 361444
(43) Date of publication of application: 05.10.2011
(62) Divisional of application: 04709922.1
(73) Proprietor: Colgate-Palmolive Company, New York NY 10022-7499 (US)
(72) Inventor: Kazmi, Perveen, Allentown, NJ New Jersey 08501 (US); Draelos, Zoe, D., High Point, NC North Carolina 27262 (US); Volz, Elizabeth, D., Princeton, NJ New Jersey 08540 (US)
(74) Representative: Jenkins, Peter David

(56) References cited:
- HARDING C R ET AL: "Dry skin, moisturization and corneodesmolysis.", INTERNATIONAL JOURNAL OF COSMETIC SCIENCE FEB 2000 LNKD- PUBMED:18503460, vol. 22, no. 1, February 2000 (2000-02), pages 21-52, XP055003607, ISSN: 1468-2494
- SIMON M ET AL: "PERSISTENCE OF BOTH PERIPHERAL AND NON-PERIPHERAL CORNEODESMOSOMES IN THE UPPER STRATUM CORNEUM OF WINTER XEROSIS SKIN VERSUS ONLY PERIPHERAL IN NORMAL SKIN", JOURNAL OF INVESTIGATIVE DERMATOLOGY, NATURE PUBLISHING GROUP, GB, vol. 116, no. 1, 1 January 2001 (2001-01-01), pages 23-30, XP001149809, ISSN: 0022-202X, DOI: DOI:10.1046/J.1523-1747.2001.00208.X
- CORCUFF, P. ET AL.: "Quantitative aspect of corneocytes", JOURNAL OF THE SOCIETY OF COSMETIC CHEMISTS, vol. 33, no. 1, 1 January 1982 (1982-01-01), pages 1-7, XP002653409,
- UHODA EMMANUELLE ET AL: "Skin weathering and ashiness in black Africans.", EUROPEAN JOURNAL OF DERMATOLOGY : EJD 2003 NOV-DEC LNKD- PUBMED:14721778, vol. 13, no. 6, November 2003 (2003-11), pages 574-578, XP002653410, ISSN: 1167-1122

## Description

### METHOD OF USE

### BACKGROUND OF THE INVENTION

Dry skin is one of the most common skin conditions manifested by persons of both sexes and all ages. Dry skin is initially observed as an increase in the number of desquamated corneocytes present on the skin surface. These corneocytes appear as white dander on the skin of Caucasian individuals, but do not represent a significant cosmetic concern, since the background skin color is also light. This is not the case in persons with darker colored skin types, such as Fitzpatrick types 4 or higher. Dry skin in darker pigmented individuals is known as ashen skin, since the pigmented skin scale appears gray, due to the presence of increased melanin in the desquamating corneocytes. Dry ashen skin, medically known as xerosis, is the result of decreased water content of the stratum corneum, which leads to abnormal desquamation of corneocytes.

A new method has been discovered which provides a non-invasive, objective measurement of corneocyte scale of people with xerosis. Compositions for treating people with xerosis can be readily evaluated using this test. In this manner, a composition has been found which effectively treats people with xerosis, ashen skin of those people having a Fitzpatrick type 4 or higher. This composition is not only effective during topical application but even has a residual effect after application of the composition is withdrawn from use.

In the International Journal of Cosmetic Science, vol. 22, no. 1 (February 2000), pages 21-52, Harding C.R. et al discuss "Dry skin, moisturization and corneodesmolysis". In the Journal of Investigative Dermatology, vol. 116, no. 1 (January 2001), pages 23-30, Simon M et al discuss "Persistence of both peripheral and non-peripheral corneodesmosomes in the upper stratum corneum of winter xerosis skin versus only peripheral in normal skin". In the Journal of the Society of Cosmetic Chemists, vol. 33, no. 1 (January 1982) pages 1-7, Corcuff P et al discuss "Quantitative aspect of corneocytes".

### SUMMARY OF THE INVENTION

The present invention provides a method according to claim 1 for assessing the presence of ashen skin on an individual having xerosis and a Fitzpatrick type 4 or higher score, which method comprises scraping the skin of said individual, collecting the loose dander accumulated, and counting the number of corneocytes.

The corneocytes can be counted visually. Alternatively, the corneocytes can be counted with a microscope.

### DETAILED DESCRIPTION OF INVENTION

Ashen skin is a term used to describe the grayish white powdery appearance of the skin having darker color skin type, such as having Fitzpatrick type 4 or higher, for example black individuals. As stated previously, because of the contrast in colors, ashen skin is a significant cosmetic issue in people with dark skin. The new test system disclosed here allows a quantitative and qualitative assessment of ashen skin because the desquamating corneocytes can now be analyzed both quantitatively and qualitatively. This can be accomplished in a simple manner by scraping the skin, collecting the scrapings and visually observing and/or observing through a dissecting microscope the desquamating corneocytes in a quantitative manner and a qualitative manner. Through this procedure, the efficacy of skin formulations can now be readily assessed for their effect on skin. These formulations can be assessed for their effect on normal skin to see if the formulation brings about ashen skin or the effect of a formulation on individuals who have ashen skin. Up until now, the state of the art system for evaluating the status of ashen skin was to make visual observation of the ashen skin. The skin scraping method presently disclosed provides a more thorough qualitative examination of the corneocytes and permits an accurate quantitative counting of the corneocytes.

Compositions which can have a positive effect on xerosis can now be evaluated utilizing this new and improved system. Moisturizing systems can be evaluated under this new system. A topical formation has been found which can be utilized in the managing of ashen skin. Interestingly, not only is skin ashiness reduced but there is also a measurable reduction in skin dryness and skin roughness. With the formulations, the positive effects are maintained for at least one week after termination of the application. This shows that there is a residual effect as well. The formulation is a rinse-off skin cleansing formulation comprising (a) at least one cleansing surfactant or a mixture of cleansing surfactants in quantities sufficient to cleanse the skin, (b) a hydrocarbonaceous substance in quantities sufficient to bring about reduction of skin ashiness in individuals having xerosis, the reduction as measured by a scraping test. The composition is rinsed from the skin with water after use in a time period of less than one minute, preferably less than 45, 30 or 15 seconds after application. This is in contrast to a lotion or cream (leave on composition) which is left on the skin as long as possible.

Any cleansing sulfactant can be employed in the cleansing composition.

Cleansing surfactants which can be employed include: Soap, a long chain alkyl or alkenyl, branched or normal carboxylic acid salt such as sodium, potassium, ammonium or substituted ammonium salt, can be present in the composition as an example of an anionic surfactant. Exemplary of long chain alkyl or alkenyl are from about 8 to about 22 carbon atoms in length, specifically about 10 to about 20 carbon atoms in length, more specifically alkyl and most specifically normal, or normal with little branching. Small quantities of olefinic bond(s) may be present in the predominantly alkyl sections, particularly if the source of the "alkyl" group is obtained from a natural product such as tallow, coconut oil and the like.

Other cleansing surfactants can be present in the composition as well. Examples of such surfactants are the anionic, amphoteric, nonionic and cationic surfactants. Examples of anionic surfactants include but are not limited to soaps, alkyl sulfates, anionic acyl sarcosinates, methyl acyl taurates, N-acyl glutamates, acyl isethionates, alkyl sulfosuccinates, alkyl phosphate esters, ethoxylated alkyl phosphate esters, trideceth sulfates, protein condensates, ethoxylated alkyl sulfates and the like.

Alkyl chains for these surfactants are C₈-C₂₂, preferably C₁₀-C₁₈, more preferably C₁₂-C₁₄.

Anionic non-soap surfactants can be exemplified by the alkali metal salts of organic sulfate having in their molecular structure an alkyl radical containing from about 8 to about 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols (C₈-C₁₈ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art.

Zwitterionic surfactants can be exemplified by those which can be broadly described as derivatives of aliphatic quaternary ammonium, phosphonium, and sulfonium compounds, in which the aliphatic radicals can be straight chain or branched and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is: 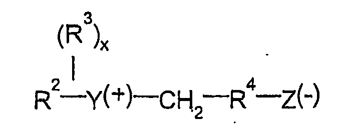 wherein R² contains an alkyl, alkenyl, or hydroxy alkyl radical of from about 8 to about 18 carbon atoms, from 0 to about 10 ethylene oxide moieties and from 0 to I glyceryl moiety; Y is selected from the group consisting of nitrogen, phosphorus, and sulfur atoms; R3 is an alkyl or monohydroxyalkyl group containing 1 to about 3 carbon atoms; X is I when Y is a sulfur atom and 2 when Y is a nitrogen or phosphorus atom, R⁴ is an alkylene or hydroxyalkylene of from 0 to about 4 carbon atoms and Z is a radical selected from the group consisting of carboxylate, sulfonate, sulfate, phosphonate, and phosphate groups.

Examples include: 4-[N,N-di(2-hydroxyethyl)-N-octadecylammonio]-butane-1-carboxylate; 5-[S-3-hydroxypropyl-S-hexadecylsulfonio] -3 hydroxypentane-1-sulfate; 3-[P,P-P-diethyf-P 3,6,9 trioxatetradecyl- phosphonio]-2-hydroxypropane-1-phosphate; 3-[N,N-dipropyl-N-3 dodecoxy-2-hydroxypropylammonio]-propane-I-phosphonate; 3-(N,N-di- methyl-N-hexadecylammonio) propane-1-sulfonate; 3-(N,N-dimethyl-N-hexadecylammonio)-2-hydroxypropane-1-sulfonate; 4-(N,N-di(2-hydroxyethyl)-N-(2 hydroxydodecyl) ammonio]-butane-1-carboxylate; 3-[S-ethyl-S-(3-dodecoxy-2-hydroxypropyl)sulfonio]-propane-1-phosphate; 3-(P,P-dimethyl-P-dodecylphosphonio)-propane-1-phosphonate; and 5-[N,N-di(3-hydroxypropyl)-N-hexadecylammonio]-2-hydroxy-pentane-1-sulfate.

Examples of amphoteric surfactants which can be used in the compositions are those which can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate; sulfate, phosphate, or phosphonate. Examples of compounds falling within this definition are sodium 3-dodecylaminopropionate, sodium 3-dodecylaminopropane sulfonate, N-alkyltaurines, such as the one prepared by reacting dodecylamine with sodium isethionate according to the teaching of U.S. Patent No.2,658,072 , N-higher alkyl aspartic acids, such as those produced according to the teaching of U.S. Patent No. 2,438,091 , and the products sold under the trade name "Miranol" and described in U.S. Patent No. 2,528,378 . Other amphoterics such as betaines are also useful in the composition.

Examples of betaines useful herein include the high alkyl betaines such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl) carboxymethyl betaine, oleyl dimethyl gamma-carboxypropyl betaine, lauryl bis-(2-hydro-xypropyl) alpha-carboxyethyl betaine, etc. The sulfobetaines may be represented by coco dimethyl sulfopropyl betaine, stearyl dimethyl sulfopropyl betaine, amido betaines, amidosulfobetaines, and the like.

Many cationic surfactants are known to the art. By way of example, the following may be mentioned:
- stearyldimenthylbenzyl ammonium chloride;
- dodecyltrimethylammonium chloride;
- nonylbenzylethyldimethyl ammonium nitrate;
- tetradecylpyridinium bromide;
- laurylpyridinium chloride;
- cetylpyridinium chloride;
- laurylpyridinium chloride;
- laurylisoquinolium bromide;
- ditallow(Hydrogenated)dimethyl ammonium chloride;
   dilauryldimethyl ammonium chloride; and
- stearalkonium chloride.

Nonionic surfactants include those which can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:
1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.
2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from about 5,000 to about 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.
3. The condensation product of aliphatic alcohols having from 8 to 18 carbon atoms, in either straight chain or branched chain configuration with ethylene oxide, e.g., a coconut alcohol ethylene oxide condensate having from 10 to 30 moles of ethylene oxide per mole of coconut alcohol, the coconut alcohol fraction having from 10 to 14 carbon atoms. Other ethylene oxide condensation products are ethoxylated fatty acid esters of polyhydric alcohols (e.g., Tween 20-polyoxyethylene (20) sorbitan monolaurate).
4. Long chain tertiary amine oxides corresponding to the following general formula:

   R₁R₂R₃N→O

   wherein R₁ contains an alkyl, alkenyl or monohydroxy alkyl radical of from about 8 to about 18 carbon atoms, from 0 to about 10 ethylene oxide moieties, and from 0 to 1 glyceryl moiety, and, R₂ and R₃ contain from 1 to about 3 carbon atoms and.from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyl-di(2-hydroxyethyl) amine oxide, dimethyloctylamine oxide, dimethyldecylamine oxide, dimethyl-tetradecylamine oxide, 3,6,9 trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethyl- hexadecylamine oxide.
5. Long chain tertiary phosphine oxides corresponding to the following general formula:

   RR'R"P→O

   wherein R contains an alkyl, alkenyl or monohydroxyalkyl radical ranging from 8 to 20 carbon atoms in chain length, from 0 to about 10 ethylene oxide moieties and from 0 to 1 glyceryl moiety and R' and R" are each alkyl or mono-hydroxyalkyl groups containing from 1 to 3 carbon atoms. The arrow in the formula is a conventional representation of a semipolar bond. Examples of suitable phosphine oxides are: dodecyldimethylphosphine oxide, tetra-decylmethylethylphosphine oxide, 3,6,9-trioxaoctadecyldimethylphosphine oxide, cetyldimethylphosphine oxide, 3-dodecoxy-2-hydrbxypropyldi(2-hydroxyethyl) phosphine oxide stearyldimethylphosphine oxide, cetylethyl propylphosphine oxide, oleyldiethylphosphine oxide, dodecyldiethylphosphine oxide, tetra-decyldiethylphosphine oxide, dodecyldipropylphosphine oxide, dodecyl-di(hydroxymethyl)phosphine oxide, dodecyldi(2-hydroxyethyl)phosphine oxide, tetradecylmethyl-2-hydroxypropylphosphine oxide, oleyldimethylphosphine oxide, 2-hydroxydodecyldimethylphosphine oxide.
6. Long chain dialkyl sulfoxides containing one short chain alkyl or hydroxy alkyl radical of 1 to about 3 carbon atoms (usually methyl) and one long hydrophobic chain which contain alkyl, alkenyl, hydroxy alkyl, or keto alkyl radicals containing from about 8 to about 20 carbon atoms, from 0 to about 10 ethylene oxide moieties and from 0 to 1 glyceryl moiety. Examples include: octadecyl methyl sulfoxide, 2-ketotridecyl methyl sulfoxide, 3,6,9-trioxaoctadecyl 2-hydroxyethyl sulfoxide, dodecyl methyl sulfoxide, oleyl 3-hydroxypropyl sulfoxide, tetradecyl methyl sulfoxide, 3 methoxytridecylmethyl sulfoxide, 3-hydroxytridecyl methyl sulfoxide, 3-hydroxy-4-dodecoxybutyl methyl sulfoxide.
7. Alkyl polyglycosides wherein the alkyl group is about 10 to about 20 carbon atoms, preferably about 10 to about 18 carbon atoms and the degree of polymerization of the glycoside is from about 1 to about 3, preferably about 1.3 to about 2.0.

Since dry ashen skin, xerosis, seems to be the result of decreased water content of the stratum corneum which leads to abnormal desquamation of corneocytes, the presence of a hydrocarbonaceous substance seems to bring alleviation of the problem through moisturization of the skin. Substances which bring about reduction of skin ashiness include long chain alkanes, long chain alkenes, mineral oils, petrolatum, polyalpha olefins, microcrystalline waxes, paraffin wax, beeswax and natural waxes derived from vegetables and mixtures thereof.

Other materials can also be in the composition as well. Examples of these components are coloring agents, UV stabilizers, antibacterial agents, thickeners, gelling agents, additional emollients and the like. Preferred components for reasons including as additional skin feel include cationic polymers. Examples of such cationic materials include the various polyquats known to the art which include but are not limited to Polyquaternium 2 (a polyelectrolyte formed from quaternized ioenes), Polyquaternium 4 (hydroxycellulose diallyldimethyl ammonium chloride), Polyquaternium 5 (acrylamide/β--methacryloxyethyltrimethyl ammonium methosulfate), Polyquaternium 6 and 7 (homopolymer of dimethyl diallyl ammonium chloride and the copolymer of dimethyl diallyl ammonium chloride with acrylamide), Polyquaternium 8 (methyl and stearyl dimethylaminoethyl methacrylate quaternized with dimethylsulfate), Polyquaternium 10 (1-hydroxypropyl trimethyl ammonium chloride ethers of hydroxyethyl cellulose), Polyquaternium 11 (quaternized PVP and dimethylaminoethyl methacrylate), Polyquaternium 16 (copolymer of PVP/methyl vinylimidazoline), Polyquaternium 17 and 18 (polyelectrolyte formed from quaternized ioenes), Polyquaternium 19 (a vinyl alcohol hydroxypropyl amine salt), Polyquaternium 24 (polymeric quaternized ammonium salt of hydroxymethylcellulose and lauryl dimethyl ammonium substituted epoxide), Polyquaternium 27 (polyelectrolyte formed from quaternized ioenes). Other cationic materials include Polycare 133 (a polymethyacrylamideopropyl trimonium chloride from Rhone-Poulenc). These materials are known to be the type that neutralizes a negative charge, such as on skin:

Further emollients include long chain saturated or unsaturated fatty acids such as lauric, oleic; myristic, palmitic and stearic, cocoa butter, shea butter, lanolin, and silicones such as polydimethyl siloxane.

The physical form of the composition can be a solid such as a bar, a liquid or gel or a mousse, foam, aerosol or spray. The active portion of the aerosol spray , foam or mousse is generally a liquid or gel which has a separate delivery system. In a solid, the amount of cleansing surfactant is generally at least about 40, 50, or 60 wt. % with a maximum of no more than about 80, 85 or 92 wt. % of the composition. Generally, these are in the shape of a bar for skin cleansing. For the liquid or gel, a minimum of the skin cleansing surfactant or mixtures of surfactants is about 4 wt. % of liquid or gel composition. Preferred minimums are about 6, 8, or 10 wt. %. For the maximum quantities, there is generally less than about 35, 25, 20 or 15 wt. %.

With respect to additional emollients when employed, there is a minimum of about 0.1 wt. %, 0.2, 0.4 or 0.5 wt. % of a solid composition and a maximum or no more than about 8, 10 or 12 wt. % of the composition. With respect to a liquid or gel there is a minimum of about 0.05 wt. %, preferably about 0.1, 0.2, or 0.3 wt. %. A maximum of no more than about 25, 20, 15 or 10 wt. % can be employed.

Following is a description of the test method and the results using a soap bar having the following composition:

| **Component** | **Wt.%** |
|---|---|
| Soap | 78 |
| Petrolatum | 3.5 |
| Silicone | 0.1 |
| Polyquat 6 | 0.12 |
| Fragrance | 1.0 |
| Antioxidants, Preservatives, etc. | 2.7 |
| Water, q.s. | |

Forty male or female subjects above the age of consent with Fitzpatrick skin types 4 or higher were enrolled in a 2-week study to determine the effect of a novel soap formulation on the reduction of the appearance of ashen skin. The subjects were soap users and possessed ashen skin indicating skin dryness, but no evidence of dermatitis or other skin disease. Subjects underwent a baseline skin assessment by an investigator, skin scrapings (described below) and conducted self skin assessments of ashen skin, roughness and dryness (Table 1). Skin scrapings were used to visually assess the amount of skin scales present.

**Table 1**

| Assessment Scale for Ashen Skin, Roughness and Dryness | |
|---|---|
| **Score** | **Assessment Definition** |
| 0 | None |
| 1 | Mild Level |
| 2 | Moderate Level |
| 3 | Severe Level |

All forty subjects were given the soap bar to use for one week. After one week of use, thirty (30) subjects remained on the above bar while ten (10) subjects switched back to their normal soap. The subjects were randomly-assigned 10 subjects to Group A and 30 to Group B. In order to keep the study blinded, the medical investigator did not have knowledge of subject placement into the groups until the end of the study. Subjects returned once a week at the end of Weeks 1 and 2 for evaluation of their skin condition.

Skin scrapings were used to visually assess the amount of skin scale present. The side of a microscope slide was gently stroked across the skin of the inner right forearm (3 cm below the antecubital fossa) ten times to collect loose dander. The number of corneocytes obtained was recorded, and the quality of the skin scale was assessed with the aid of a dissecting microscope. This provided a noninvasive, objective measurement of the amount of scale present on the skin surface.

### Statistical Analysis

A paired t-test was used to assess the skin scrapings investigator and patient data for Baseline and Week 1 comparisons. A paired t-test was used to compare the Baseline and Week 2 data for the group of 30 subjects. In addition, a 2 sample unequal t-test was used when comparing the Group of 30 to the Group of 10 at the end of Week 2.

### Results and Discussion

### Skin Scale Scrapings

After one week of using the novel bar, there was a statistically significant reduction in the number of skin scales on the microscope slide (See Table 2).

**Table 2**

| Week 1 | | |
|---|---|---|
| Reduction in Skin Scales for Novel Soap Bar | | |
| **Average Cell Count** | **Baseline** n = 40 18.88 ± 2.05 | **Week 1** n = 40 11.50 ± 1.56 |
| **Paired t-Test vs. Baseline** | | <0.001 |

For Week 2, the number of corneocytes continued to decrease for those remaining on the test bar (Table 3).

**Table 3**

| Week 2 | | |
|---|---|---|
| Reduction in Skin Scales for Novel Soap Bar | | |
| **Average Cell Count** | **Baseline** n = 30 12.03 ± 1.56 | **Week 1** n = 30 6.90 ± 0.81 |
| **Paired t-Test vs. Baseline** | | <0,001 |

For the subjects that returned to their normal product. there was an increase in skin scales (Table 4).

**Table 4**

| Skin Scales Comparison at Week 2 | | |
|---|---|---|
| **Test Product** | **Novel Bar** | **Normal Bar** |
| **Assessment Parameter** | **Skin Scales** | **Skin Scales** |
| **Number of Subjects** | n = 30 | n = 10 |
| **Average Cell Count** | 6.90 ± 0.81 | 13.80 ± 1.32 |
| **2-Sample Unequal t-Test** | | 0.003 |

However, the counts at Week 2 were still lower than baseline and greater than the number collected from the users of the test soap bar. This would indicate that there was a residual benefit of the test bar even after subjects resumed use of their normal bar.

Counts were made of the larger flakes on the glass slide, ignoring debris from skin scale fragments, hairs, clothing, etc. At baseline, subjects demonstrated large cohesive skin scale flakes. These large flakes probably accounted for the appearance of ashen skin present at study entry. At the end of Week 1, not only was there reductions in the number of cell scales but the skin scales were reduced to a fine powder. The smaller size of the corneocyte debris probably accounted for the visible improvement in ashen skin. It is important to note that the group that continued to use the test soap for Week 2 demonstrated a further reduction in skin scales (Table 3).

### Skin Ashiness

As observed by the medical investigator's and subject's self-assessments, there was a statistically significant reduction from baseline in the observed level of skin ashiness at the end of Week 1. The 30 subjects that remained on the test soap bar continued to show a reduction in their ashiness levels for Week 2 as assessed by the medical investigator and subjects. The 10 subjects that returned to using their normal product for Week 2 showed an increase in skin ashiness as compared to Week 1. The subject's self-assessment indicates a return to their baseline level of ashiness. However, the medical investigator's assessment shows an increase for Week 2 but not to the baseline levels. Statistical comparison showed that at Week 2 there was significantly less ashiness in those subjects that remained on the test soap bar than those.that returned to their normal product.

### Skin Dryness

For both Weeks 1 and 2 there was a statistically significant reduction from baseline in the overall level of skin dryness as assessed by the medical investigator and the subject's self-assessments for the noel soap bar users. The dryness levels continued to decrease for those subjects that remained on the novel soap bar. The development of dryness for Week 2 follows the same pattern that was observed for ashiness by both the medical investigator and the subjects: Increase in dryness upon returning to their normal product. However, the subjects' self-assessment showed skin dryness level increased to the baseline level. At week 2, a 2-sample unequal t-test analysis indicated significantly less dryness in subjects that continued to use the novel soap bar than those that returned to their normal product.

### Skin Roughness

Assessment for skin roughness approximated the same results and had the same general trends as for skin dryness.

## Claims

1. A method for assessing the presence of ashen skin on an individual having xerosis and a Fitzpatrick type 4 or higher score which comprises:
(a) scraping the skin of said individual,
(b) collecting the loose dander accumulated, and
(c) counting the number of corneocytes.

2. The method in accordance with claim 1 wherein the corneocytes are counted visually.

3. The method in accordance with claim 1 wherein the corneocytes are counted with a microscope.

## Patentansprüche

1. Verfahren zur Detektion von Aschehaut auf einem Individuum mit einer Xerose und einem Fitzpatrick-Wert Typ 4 oder höher, wobei:
(a) dem Individuum Haut abgekratzt wird,
(b) die akkumulierten, gelösten Hautteilchen gesammelt werden und
(c) die Anzahl der Hornzellen ermittelt wird.

2. Verfahren nach Anspruch 1, wobei die Hornzellen visuell gezählt werden.

3. Verfahren nach Anspruch 1, wobei die Hornzellen mit einem Mikroskop gezählt werden.

## Revendications

1. Procédé destiné à détecter la présence d'une peau cendrée chez un individu atteint de xérosis et présentant un phototype 4 ou plus selon la classification de Fitzpatrick, et qui consiste à :
(a) racler la peau dudit individu,
(b) recueillir les produits de desquamation accumulés, et
(c) compter le nombre de cornéocytes.

2. Procédé selon la revendication 1, dans lequel les cornéocytes sont comptés visuellement.

3. Procédé selon la revendication 1, dans lequel les cornéocytes sont comptés à l'aide d'un microscope.
